# EUROPEAN PATENT APPLICATION

(11) **EP 1 438 980 A2**
(43) Date of publication of application: **21.07.2004**
(21) Application number: 04008352.9
(22) Date of filing: 13.06.1997
(51) Int. Cl.: A61M 25/01, A61M 16/04, A61B 5/06, G01V 3/08

(54) **Medical tube for insertion and detection within the body of a patient**

(30) Priority: 17.06.1996 US 664501
(62) Divisional of application: 97931176.8
(71) Applicant: Becton, Dickinson and Company, Franklin Lakes, New Jersey 07417 (US)
(72) Inventor: The designation of the inventor has not yet been filed
(74) Representative: Hoarton, Lloyd Douglas Charles

(57) **Abstract**

There is disclosed a medical tube for insertion into the body of a patient. The medical tube includes a tube or device suitable for insertion into the patient's body, and a permanent magnet associated therewith. The magnet may be solid or non-solid, and may be rigid or non-rigid. In one embodiment, the magnet is hollow and associated with the medical tube such that the tube may be used for its intended purposes. In another embodiment, the magnet is solid and, after insertion into the body of the patient, is displaced such that it does not interfere with the intended use of the medical tube. In a further embodiment, the magnet is removable after placement of the medical tube.

## Description

### Technical Field

This invention is generally directed to a medical tube for insertion and detection within the body of a patient.

### Background of the Invention

There are many instances in clinical medicine where detecting the location of a medical tube within a patient is important. For example, when positioning feeding tubes through the mouth or nose of a patient, it is essential that the end of the feeding tube pass into the patient's stomach, and that it does not "curl up" and remain in the esophagus. For example, if the end of the feeding tube is improperly positioned in the trachea rather than stomach, aspiration of the feeding solution into the patient's lungs may occur.

In addition to feeding tubes, a variety of other medical tubes require accurate positioning within a patient's body, including dilating tubes to widen an esophageal stricture, tubes for measuring pressure waves in the stomach and esophagus of a patient who is suspected of having esophageal motor disorders, Sengstaken-Blakemore tubes in the stomach and esophagus of a patient to control bleeding from varicose veins in the esophagus, colonic decompression tubes in the colon of a patient to assist in relieving distention of the colon by gas, urologic tubes in the bladder, ureter or kidney of a patient, and vascular tubes in the heart or pulmonary arteries of a patient. In fact, any catheter with a tip inserted in the body of a patient (via the mouth, anus, urethra, etc.), or between any two structures in the body (such as a stent) generally require accurate positioning.

Currently, the location of a medical tube within the body of a patient is routinely detected by the use of imaging equipment, such as a chest or abdominal X-ray. However, such a procedure requires transportation of the patient to an X-ray facility or, conversely, transportation of the X-ray equipment to the patient. This is both inconvenient and costly to the patient, and is particularly stressful in those instances where the patient repeatedly and inadvertently removes a medical tube, such as a feeding tube, thus requiring repeated reinsertion and X-rays. X-ray guidance also takes considerable time, making it inconvenient for multiple repositioning of a tube when the patient pulls on the tube or when the care-giver repositions the tube.

Recently, U.S. Patent No. 5,425,382 to Golden et al. discloses a detection apparatus for detecting the location of a medical tube within the body of a patient. That detection apparatus senses the static magnetic field strength gradient generated by a magnet associated with the medical tube, and indicates the value and magnitude of the gradient to the user. Use of such a detection apparatus allows rapid detection and verification of medical tube placement, and does not require that placement of the medical tube be confirmed with an X-ray.

Despite the advances made in this field, there still exists a need in the art for additional and/or improved medical tubes capable of being detected by a suitable detection apparatus. The present invention fulfills that need, and provides further related advantages.

### Summary of the Invention

In brief, this invention discloses medical tubes capable of being detected and located within the body of patient by a suitable detection apparatus. The medical tubes of the present invention comprise a tube or device suitable for insertion into the body of a patient, and include a permanent magnet associated therewith. The magnet may be a solid or non-solid (*e.g.*, hollow) magnet, and may further be a rigid or non-rigid (*e.g.,* malleable) magnet. In preferred embodiments, the magnet is either permanently or removably associated with the tube.

In one embodiment of this invention, a medical tube is disclosed comprising a tube or device suitable for insertion into a patient's body and a hollow permanent magnet associated with the medical tube. The hollow magnet may be associated with an external surface or internal surface of the medical tube, or may be integral to the medical tube itself.

In another embodiment, a medical tube is disclosed comprising a tube or device suitable for insertion into a patient's body and a permanent magnet associated with the medical tube, wherein the magnet is associated with the medical tube at a first position. Upon insertion into the patient's body, the magnet is capable of being displaced to a second position that does not interfere with the intended use of the medical tube.

In yet a further embodiment, a medical tube is disclosed comprising a tube or device suitable for insertion into a patient's body and a permanent magnet associated with the medical tube, wherein the magnet is associated with an external surface of the medical tube.

In still a further embodiment, a medical tube is disclosed comprising a tube or device suitable for insertion into a patient's body, and further includes both a permanent magnet and sensing element or device associated with the medical tube.

These and other aspects of the present invention will be better understood upon reference to the following detailed description and accompanying drawings.

### Brief Description of the Drawings

Figures 1A through 1F illustrate representative magnets which may be associated with a medical tube of this invention.
Figures 2A through 2D illustrate representative configurations for associating a hollow magnet with a medical tube.
Figures 3A and 3B illustrate a magnet associated with an interior surface (Figure 3A) and an exterior surface (Figure 3B) of a medical tube.
Figures 4A through 4C illustrate a medical tube having a magnet associated therewith, and wherein the magnet is capable of being displaced to a non-interfering position after insertion of the medical tube into the body of a patient.
Figures 5A and 5B illustrate an alternative embodiment where the magnet is capable of being displaced to a non-interfering position after insertion of the medical tube into the body of a patient.
Figures 6A and 6B illustrate a medical tube having a magnet associated with an external surface of the medical tube (Figure 6A), and associated with the distal end of the medical tube (Figure 6B).
Figures 7A and 7B illustrate a medical tube having a magnet associated therewith and having a biopsy port; and Figure 7C illustrates a medical tube having a magnet associated therewith and having a sensing element or device located adjacent to the magnet.
Figures 8A and 8B illustrate the static magnetic field strength of a magnet associated with a medical tube of this invention. Specifically, the direction of the sensed dipole (*i.e.,* polarity) depends on the orientation of the magnet: Figure 8A depicts the magnet dipole pointing to the proximal end; and Figure 8B depicts the magnetic dipole pointing to the distal end.
Figures 9A and 9B illustrate detecting the location and orientation of a medical tube of this invention by a suitable detection apparatus.
Figure 10 illustrates a representative medical tube of this invention, wherein the medical tube is a tracheal tube having a magnet associated a fixed distance from the distal end.

### Detailed Description of the Invention

As indicated above, the present invention is directed to medical tubes for insertion into and detection within the body of a patient. As used herein, the term "medical tube" means any and all types of tubes or devices which may be inserted into a patient's body, including (but not limited to) catheters, guide wires, stents, shunts and medical instruments. As used herein, "catheters" include such items as feeding tubes, urinary catheters and dilating catheters, as well as nasogastric tubes, endotracheal tubes, stomach pump tubes, wound drain tubes, rectal tubes, vascular tubes, Sengstaken-Blakemore tubes, colonic decompression tubes, pH catheters, blood-gas sensors, pressure tubes, image capture tubes, motility catheters, and urological tubes. "Guide wires" are often used to guide or place dilators and other medical tubes within the body of a patient, and are considered medical tubes within the context of this invention. "Medical instruments" include endoscopes and colonoscopes, as well as imaging equipment such as video and ultrasound imaging equipment, and are considered medical tubes as the term is used herein. In short, as used in the context of this invention, the term medical tube is intended to encompass any foreign object that may be inserted into a patient's body for any purpose, including (but not limited to) medical, diagnostic and/or therapeutic purposes.

Once inserted into the body of the patient, the medical tube is detected by a suitable detection apparatus. In the practice of this invention, a preferred detection apparatus is that disclosed in U.S. Patent No. 5,425,382 and International Application No. PCT/US94/10417 which published as International Publication No. WO 95/08130 on March 23, 1995, both to Golden et al. (which documents are incorporated herein by reference in their entirety, and collectively referred to as "the Golden et al. detection apparatus"). The Golden et al. detection apparatus detects both location and orientation of a medical tube by sensing a static magnetic field strength gradient produced by a permanent magnet associated with the medical tube. As used herein, the term "associated with" means permanently affixed, removably attached, or in close proximity to, the medical tube.

In one embodiment, such as a feeding tube, the magnet may be associated with the distal end of the medical tube. In other embodiments, the magnet may be associated with the medical tube at a location between its proximal and distal ends. For example, in the case of a Sengstaken-Blakemore tube, the magnet may be associated with the medical tube at a location above the gastric balloon and below the esophageal balloon. In the case of endotracheal or nasotrachial tubes, the magnet may be positioned at a specific distance from the distal end of the tube as discussed in greater detail below.

Since the magnet of this invention is permanent, it requires no power source. Accordingly, the magnet maintains its magnetic field indefinitely, which allows long-term positioning and detection of the medical tube without the disadvantages associated with an internal or external power source. In particular, by avoiding the use of a power source, the undesirable electrical connections necessary for the use of a power source are avoided. Thus, there is no risk of shock to (or possible electrocution of) the patient due to the magnet. Furthermore, the magnet's static magnetic field passes unattenuated through body tissue and bone. This property allows detection of the medical tube at any location within the patient's body.

As mentioned above, the magnet (and hence the medical tube) may be detected using any suitable detection apparatus. In a preferred embodiment, the detection apparatus is the Golden et al. detection apparatus. That detection apparatus contains two static magnetic field strength sensors configured geometrically to null detection of ambient, homogeneous magnetic fields (*e.g.*, the earth's field), while still detecting the magnetic field strength gradient produced by the magnet associated with the medical tube. The detection apparatus is an active, electronic instrument, and can detect the relatively small magnetic field strength gradient produced by the magnet at distances ranging from several centimeters to several decimeters, and preferably from about 2 centimeters to about 3 decimeters. It also indicates the value of the gradient, thus allowing the user to accurately determine the location and orientation of the magnet, and hence the medical tube. In a preferred embodiment, the detection apparatus indicates the value of the gradient as both a magnitude and a polarity.

Due to the sensitivity of the Golden et al. detection apparatus to the magnet's field strength gradient, additional imaging equipment is not necessary to confirm the location of the medical tube after insertion. Accordingly, the medical tubes of this invention are suitable for use in environments which lack such equipment. For example, nursing homes rarely have X-ray equipment on-site, and the medical tubes of the present invention are particularly suited for use in such facilities. Other useful settings include emergency rooms where quick insertion and immediate use of medical tubes are often necessary. In addition, the use of medical tubes of this invention would reduce X-ray exposure during fluoroscopy. Alternatively, after X-ray verification of the initial placement of the medical tube, its location thereafter can be verified with the detection apparatus, thus avoiding subsequent X-rays to confirm its location.

As mentioned above, the Golden et al. detection apparatus detects both location and orientation of the magnet associated with the medical tube. In other words, that detection apparatus indicates to the user the direction of the magnet's dipole. Thus, by associating the magnet with the medical tube in a fixed and known orientation, the orientation of the magnet (and hence the medical tube) can be determined. In a preferred embodiment, the magnet is associated with the medical tube such that it's dipole is parallel to the longitudinal axis of the medical tube (*i.e.*, the axis extending from the proximal end to distal end of the medical tube) and, in a more preferred embodiment, is parallel to the longitudinal axis of the medical tube and pointing toward the proximal end (*i.e.*, the north pole of the magnet is nearer the proximal end of the medical tube than the south pole).

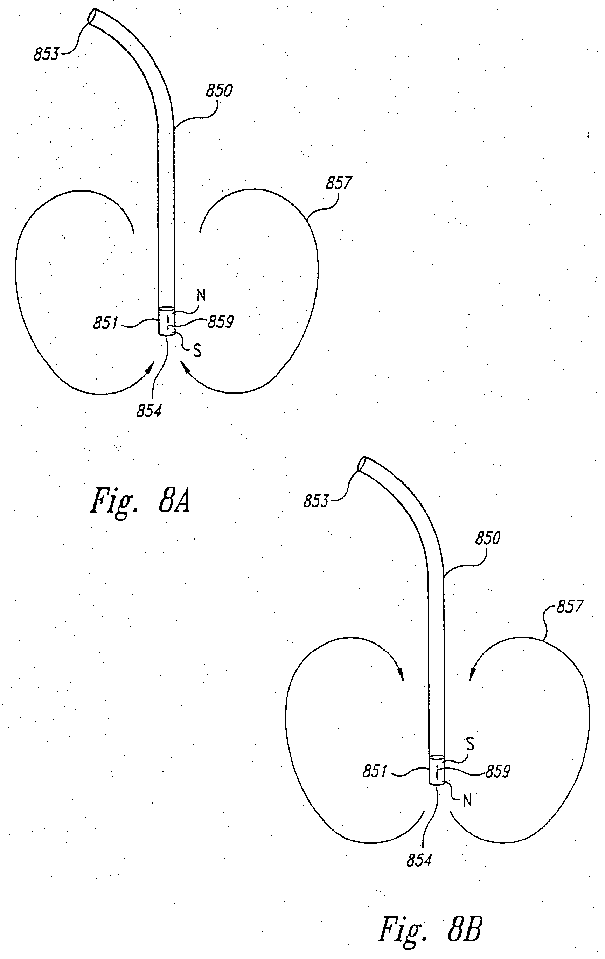

In addition, it should be recognized that, when detecting the magnet with the Golden et al. detection apparatus, the polarity of the value of the differential signal (*i.e.*, positive or negative) depends on the orientation of the sensed magnet. As illustrated in Figures 8A and 8B, medical tube (850) having a proximal end (853) and distal end (854), has magnet (851) associated with the distal end of the tube. Lines (857) represent the static magnetic field produced by the magnet, the direction of which depends on whether the magnet is associated with the tube such that its dipole, represented by arrow (859), points parallel to the longitudinal axis of the tube in the direction of the proximal end (Figure 8A) or in the direction of the distal end (Figure 8B).

Since the value of the differential signal indicates the direction of the magnet, the dipole of the magnet is associated with the medical tube in a fixed and known orientation. Furthermore, the detection apparatus is calibrated such that the orientation of the magnet (and hence the medical tube) is properly displayed to the user. The medical tubes of this invention preferably have the magnet affixed thereto as represented in Figure 8A -- that is, with the magnet's dipole pointing parallel to the longitudinal axis of the medical tube in the direction of the proximal end. By maintaining this orientation, the Golden et al. detection apparatus can indicate the orientation of the magnet (which, in the preferred embodiment, is 180 degrees from the direction of the magnetic dipole). This feature is useful in a variety of settings. For example, when placed at the end of a feeding tube, the Golden et al. detection apparatus indicates to the user whether the distal end of the feeding tube is pointing towards the patient's feet, or towards the patient's head. If pointing towards the patient's head, this could indicate that the tube is improperly inserted. Similarly, in the case of medical tubes such as guide wires, the user can determine in which direction the guide wire is traveling to confirm that it has, for example, entered the desired artery and is traveling in the desired direction.

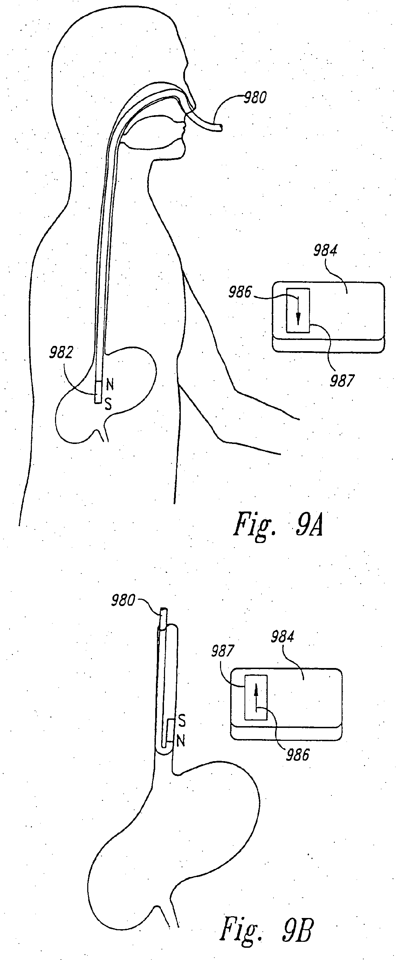

This aspect of the present invention is further illustrated by reference to Figures 9A and 9B. Figure 9A depicts a feeding tube (980), with permanent magnet (982) associated with the distal end of the medical tube, and wherein the magnetic dipole of the magnet points toward the proximal end of the medical tube. After insertion into the patient's stomach, detection apparatus (984) detects the location of the magnet and, as indicated by arrow (986) in visual display (987), properly indicates to the user the orientation of the magnet, and hence the orientation of the distal end of the medical tube. For example, had feeding tube (980) "curled up" in the esophagus as illustrated in Figure 9B, arrow (986) in visual display (987) of detection apparatus (984) would indicate that the distal end of the medical tube is pointing toward the patient's head, and thus the feeding tube is not correctly positioned within the patient.

The magnet of this invention is generally a relatively small, rare-earth magnet. Suitable magnets include rare earth magnets such as samarium cobalt and neodymium iron boron, both of which generate high field strengths per unit volume. While magnets which generate a high field strength for their size are preferred, weaker magnets such as Alnico or ceramic may also be utilized. As discussed in greater detail below, the magnets of this invention may be solid or non-solid magnets, and may further be rigid or non-rigid magnets. Non-rigid magnets include (but are not limited to) suspensions of magnetic particles, as well as malleable forms of magnetic material (such as a putty).

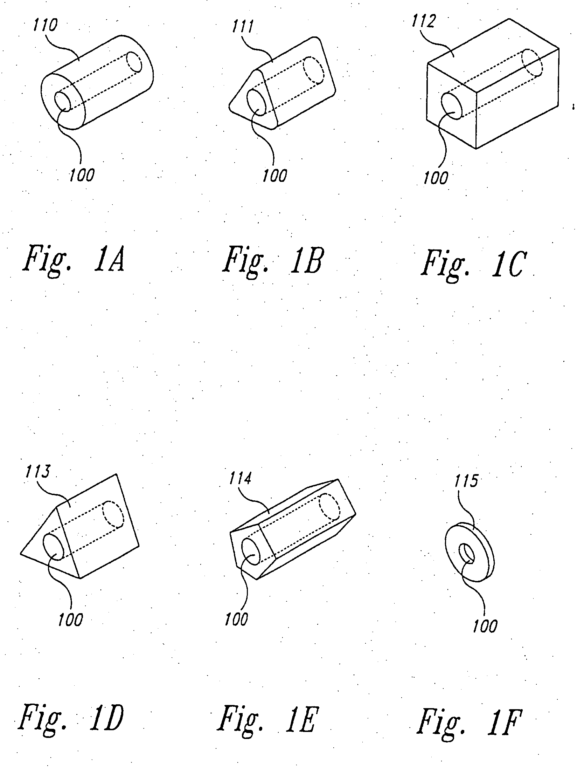

In one embodiment, the magnet is a non-solid, hollow magnet having an interior chamber. Representative embodiments of hollow magnets suitable for use in the practice of this invention are illustrated in Figure 1. Referring to Figure 1A, hollow cylindrical magnet (110) is illustrated, having interior chamber (100). Similarly, Figures 1B, 1C, 1D and 1E illustrate hollow ellopsoid magnet (111), hollow rectangular magnet (112), hollow prism magnet (113) and hollow polygon magnet (114), respectively, each having interior chamber (100). The representative hollow magnets illustrated in Figures 1A through 1E typically have a length ranging from about 0.75 mm to about 12 mm, and preferably from 1.5 mm to 6 mm.

Alternatively, the length of the hollow magnet may be relatively short, yielding a thin magnet. For example, the hollow cylindrical magnet of Figure 1A may be in the form of hollow torrus or ring magnet (115) as illustrated in Figure 1F, and having interior chamber (100). Other configurations may similarly be employed. In this embodiment, the hollow ring magnet may have a length or thickness typically ranging from about 0.1 mm to about 5 mm.

In the practice of this invention, a single magnet or multiple magnets may be associated with a single medical tube. For example, in the case of thin magnets, such as the hollow ring magnet illustrated in Figure 1F, a plurality of hollow ring magnets may be associated with the medical tube.

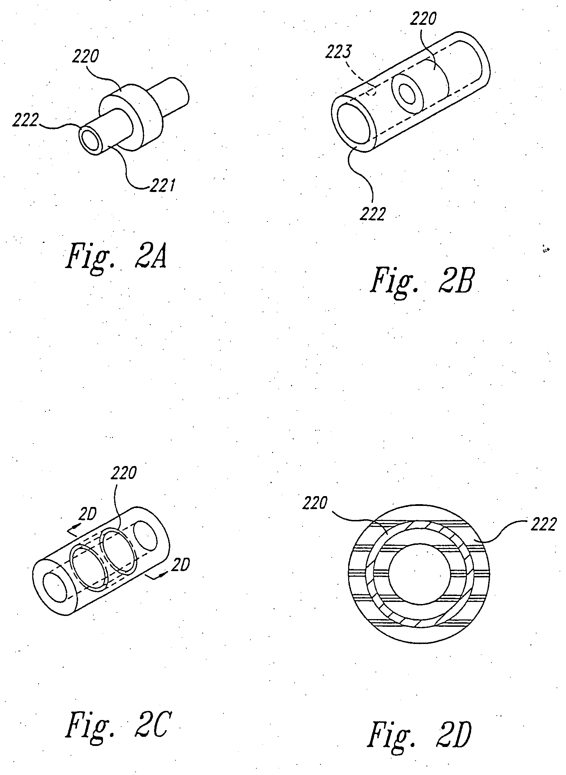

In one embodiment of this invention, the hollow magnet is associated with the medical tube such that material, light, data, etc., may pass through the interior chamber of the magnet. This may be achieved, for example, by associating the hollow magnet with the medical tube as illustrated in Figure 2. Referring to Figure 2A, hollow magnet (220) may be associated with medical tube (222) by locating the magnet around outside circumference (221) of the medical tube. Alternatively, hollow magnet (220) may be associated with interior circumference (223) of medical tube (222) as illustrated in Figure 2B, or hollow magnet (220) may be integral to medical tube (222) as illustrated in Figures 2C and 2D.

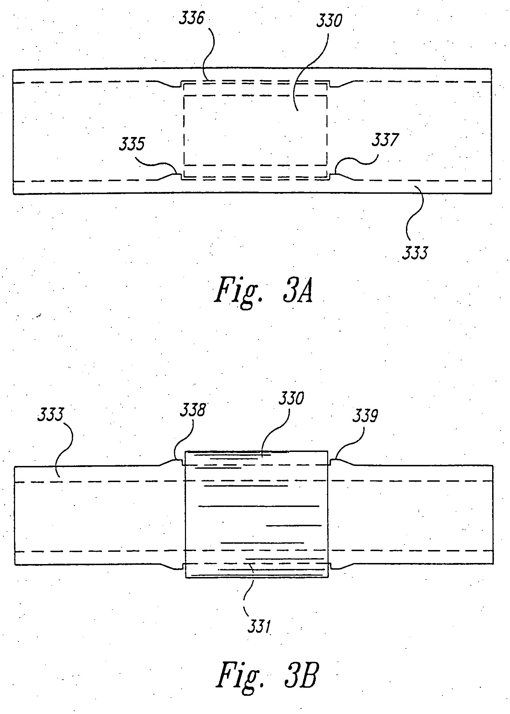

The hollow magnet may be associated with the medical tube by being affixed thereto, or may be confined to a specific location of the medical tube by, for example, locating the magnet within an appropriate magnet chamber or area. For example, referring to Figure 3A, hollow magnet (330) may be located within an internal area of medical tube (333) defined by interior surface (336) and internally protruding ribs (335) and (337). Alternatively, as illustrated in Figure 3B, hollow magnet (330) may be confined to an external area of medical tube (333) defined by exterior surface (331) and externally protruding ribs (338) and (339). In this embodiment, the length of the magnet, as well its exterior diameter in the case of Figure 3A, or its interior diameter in the case of Figure 3B, are sized such that the magnet remains associated with the medical tube between the protruding ribs.

It should be recognized, however, that a variety of techniques could be employed to associate the magnet with the medical tube. Such techniques include (but are not limited to) the use of suitable adhesives and/or tape, as well as incorporating the magnet in the manufacture of the medical tube such that it becomes integral to the tube itself.

In the above embodiments, the hollow magnet is preferably a rigid magnet, and the interior chamber is appropriately sized to permit the medical tube to be used to perform its intended function. For example, in the case of a feeding tube, the interior chamber has a sufficient volume to permit food, as well as other solid, liquid and/or gaseous materials, to pass through the interior of the feeding tube, through the interior chamber of the magnet, and exit the medical tube at or near the distal end. For other applications, the interior chamber would be appropriately sized for the intended purpose of the medical tube, including, for example, sized to permit the passage of light, video images, ultrasound energy, etc., to pass through the medical tube. For hollow cylindrical magnets having a cylindrical interior chamber, for example, the diameter of the interior chamber may typically range from about 0.5 mm to about 3 mm.

In another aspect of this invention, the magnet may be a solid magnet. In this embodiment the magnet may be associated with the medical tube at a first position which obstructs or interferes with the intended use of the medical tube, and is capable of being moved or displaced to a second position after insertion. The second position of the magnet is such that the magnet does not obstruct or interfere with the intended use of the medical tube.

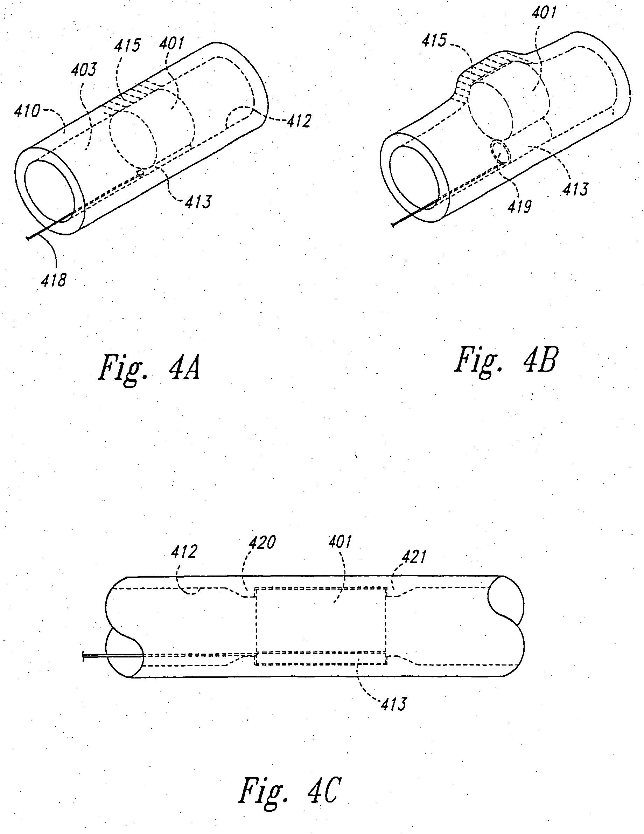

In this embodiment, the magnet may be associated within an interior channel of the medical tube. After placement of the medical tube within the patient's body, the magnet is capable of being displaced to a non-interfering position by use of an appropriate displacing device. For example, as illustrated in Figure 4A, magnet (401) is located within interior channel (403) of medical tube (410). Adjacent to the magnet, and in contact therewith, is pliable material (415) which is susceptible to deformation. Inflatable chamber (413) having inflation lumen (418) is located between interior wall (412) of the medical tube and the magnet such that, when the chamber is inflated, it displaces the magnet as illustrated in Figure 4B. Referring to Figure 4B, magnet (401) is pressed against pliable material (415) which allows for displacement of the magnet along an axis transverse to the medical tube. Inflatable chamber (413) has an interior chamber (419) which, when inflatable chamber is inflated, permits the tube to be used in its intended manner.

To prevent movement of either magnet (401) or inflatable chamber (413), interior wall (412) of the medical tube may have ribs (420) and (421) protruding inwardly from interior wall (412) of the medical tube as illustrated in Figure 4C. Referring to Figure 4A, inflation lumen (418) extends from inflatable chamber (413) to the proximal end of the medical tube. To inflate the chamber, the user may, for example, inject a suitable gas or liquid into the inflation lumen, thereby inflating the inflatable chamber and displacing the magnet as illustrated in Figure 4B.

In Figure 4, the magnet is depicted as a rigid magnet. In an alternative embodiment, the magnet may be a non-rigid magnet such that the magnet is displaced to a non-interfering position by, for example, deformation caused by the inflation of the inflatable chamber.

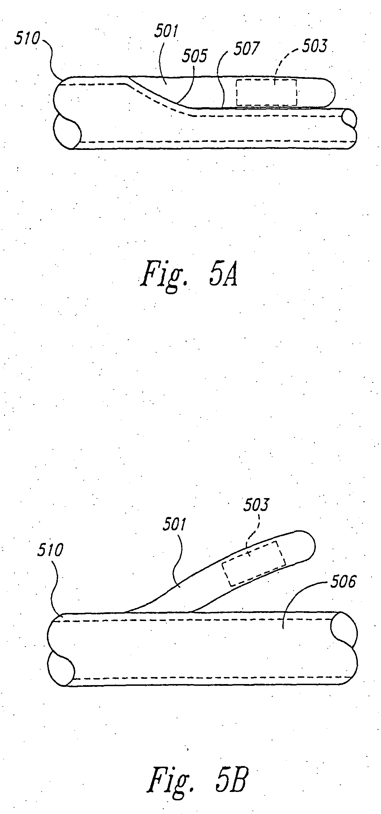

In a further alternative embodiment, the magnet may be associated with the medical tube such that, after insertion into the body of a patient, the magnet is displaced such that an interior channel of the medical tube is opened to permit the intended use of the tube. In this embodiment, the magnet may be associated with the medical tube by, for example, locating the magnet within a chamber that is attached to the medical tube. As illustrated in Figure 5A, chamber (501) contains magnet (503) and is attached to medical tube (510) along contact portion (505). For insertion into the body of the patient, the medical tube and chamber collectively assume the approximate diameter of the medical tube as illustrated in Figure 5A. This may be achieved by employing, for example, a suitable temporary adhesive along contact line (507) between chamber (501) and medical tube (510). After insertion to the desired location within the patient, magnet (503) within chamber (501) may be displaced, as illustrated in Figure 5B, to a position such that interior chamber (506) of medical tube (510) is no longer constricted, and thus the medical tube may be used for its intended purpose.

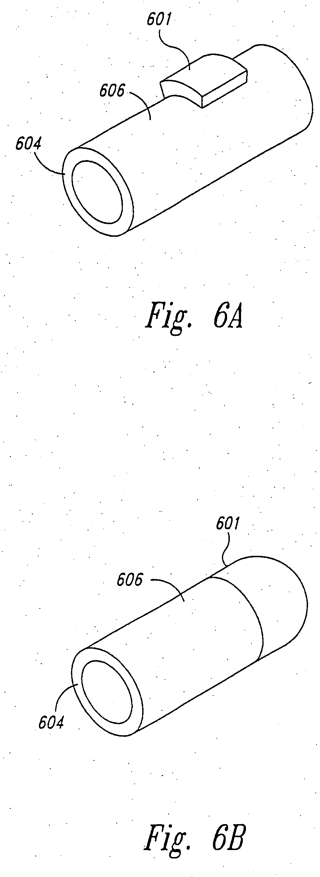

In another aspect of this invention, a medical tube is disclosed having a magnet associated with an external surface of the medical tube. As illustrated in Figure 6A, magnet (601) is associated with external surface (606) of medical tube (604). The external surface may be along the length of the medical tube, as illustrated by Figure 6A, or at the distal end, as illustrated by Figure 6B. In this embodiment, the magnet may be either a rigid or non-rigid magnet, and associated with the magnet in any suitable manner. In one embodiment, the magnet is non-rigid, and contained within a suitable enclosure associated with the medical tube. When located at the distal end of the medical tube, the magnet may be in the form of a pliable tip.

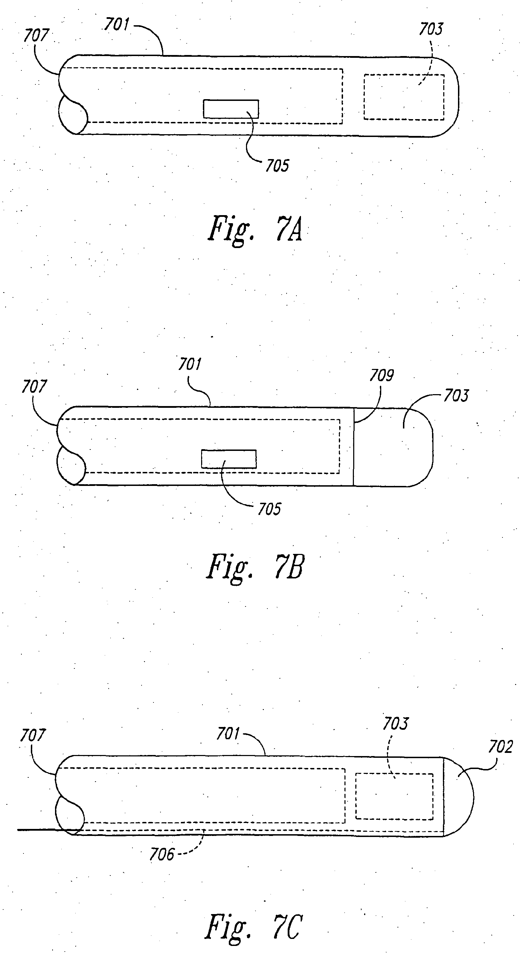

In a further embodiment, the medical tube may be a biopsy tube, wherein the magnet is associated with the biopsy tube distally to a biopsy port on the tube. Such a medical tube is illustrated in Figures 7A and 7B. Referring to Figure 7A, medical tube (701) has magnet (703) integrally associated therewith, and has biopsy port (705) located between the magnet and proximal end (707) of the medical tube. In an alternative embodiment, as illustrated in Figure 7B, medical tube (701) has magnet (703) associated with distal end (709) of the medical tube, and with biopsy port (705) located between the distal end and the proximal end (707) of the medical tube.

In another aspect of this invention, a medical tube is disclosed having a magnet associated therewith, and further comprising a sensing element or device. Suitable sensing elements and devices for use in the practice of this invention are those that sense one or more of a variety of environmental parameters, including pressure, concentration, pH, temperature, color and the like, and further include suitable devices for receiving and/or transmitting video or still images, light, ultrasound energy and the like. In this embodiment, information from the sensing element or device may be carried to the proximal end of the tube by an appropriate conduit or wire. The magnet is preferably located adjacent to the sensing element or device. A representative embodiment is illustrated in Figure 7C, where medical tube (701) has a magnet (703) associated with the medical tube and is adjacent to sensing element or device (702). An appropriate wire (or wires) (706) connect the sensing element or device to proximal end (707) of the medical tube.

As mentioned above, in the practice of this invention the magnet may be a solid or non-solid, rigid or non-rigid magnet. Rigid magnets (both solid and non-solid) are available from a variety of sources, including Dexter Corp. (Fremont, CA). Non-rigid magnets (both solid and non-solid) are generally comprised of a plurality of magnet particles contained within a suspension or slurry, or within a more solid, but malleable, substance. Suitable suspension or slurries include (but not limited to) magnetic particles within a fluid such as oil, water, glycerin, alcohol, fluid polymers and the like. More solid, yet malleable, magnets include magnetic particles within a putty, polymer, silicone, highly viscous liquid and the like. Suitable polymers include those that are solid at room temperature, but malleable at body temperature.

In the practice of this invention, non-rigid magnets are typically confined within an appropriate enclosure. In the case of suspensions or slurries, such magnets are associated with the medical tube within a suitable enclosure such that the suspension or slurry does not leak or escape from the medical tube. More viscous non-rigid magnets, such as putties and the like, are less susceptible to leakage, but may still benefit from an appropriate enclosure. Furthermore, in the case of, for example, feeding tubes, such magnets may become separated from the medical tube after some period of time and discharged in the patient's stool.

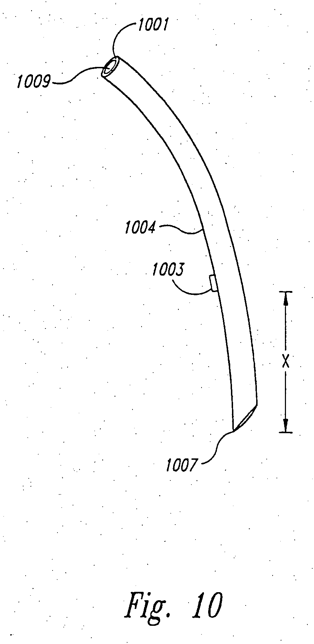

To illustrate a further representative embodiment of this invention, a tracheal tube is depicted in Figure 10. In this embodiment, the tracheal tube has a magnet associated therewith a fixed distance from the distal end. The fixed distance is such that, when the tracheal tube is properly positioned within the patient's trachea, the magnet is located immediately below (or in close proximity to) the patient's cricothyroid membrane.

A tracheal tube is typically a plastic medical tube inserted through the mouth to assist in breathing (*i.e.*, an endotracheal tube). When inserted through the nose, it is called a nasotracheal tube. As used herein, the term tracheal tube includes both endotracheal and nasotracheal tubes. Placement of the tracheal tube can be difficult, particularly with regard to depth of insertion. The tracheal tube should offer a clear ventilation path to both lungs. If inserted too deeply, the tracheal tube may direct air flow to and from only one lung, or may even block ventilation to one of the mainstem branches.

Referring to Figure 10, tracheal tube (1001) has magnet (1003) associated with exterior surface (1004) a fixed distance "X" from distal end (1007). The tracheal tube has an inner chamber (1009) which permits the passage of air through the tracheal tube. Distance "X" is such that the magnet, when the tracheal tube is properly inserted into a patient, is directly under (or in close proximity to) the patient's cricothyroid membrane. In normal adults, distance "X" may range from about 4 cm to about 6, and typically is about 5 cm.

The cricothyroid membrane is a section of tissue located between the thyroid cartilage and the cricoid cartilage. This membrane is typically 1 cm to 1½ cm below the surface of the skin in adults. The high degree of consistency from patient to patient in depth from the skin, and the ease of location of the cricothyroid membrane relative to externally identifiable landmarks on the patient make detection of the magnet at this location particularly advantageous. In this embodiment, the magnet is preferably located on the anterior side of the tracheal tube such that, when properly inserted into the patient's trachea, the magnet is immediately posterior to the cricothyroid membrane. Furthermore, the magnet dipole of the magnet may be parallel to the longitudinal axis of the tracheal tube, or transverse to the same.

From the foregoing, it will be appreciated that, although specific embodiments of this invention have been described herein for purposes of illustration, various modifications may be made without deviating from the spirit and scope of the invention. Accordingly, the invention is not limited except by the appended claims.

## Claims

1. A medical tube comprising a tube or device suitable for insertion into a patient's body and a hollow permanent magnet associated with an external surface of the medical tube or integral to the medical tube.

2. A medical tube having an intended use within a patient's body comprising a tube or device suitable for insertion into a patient's body and a permanent magnet associated with the medical tube and being displaceable with respect to the medical tube from a first position which interferes with the intended use and, after insertion into the patient's body, to a second position which does not interfere with the intended use of the tube or device.

3. A medical tube according to Claim 2, wherein means are provided to displace the magnet from the first position to the second position.

4. The medical tube of Claim 3, wherein the displacement means comprises an inflatable chamber associated with the magnet such that, upon inflation of the inflatable chamber, the magnet is displaced to the second position.

5. The medical tube of any one of Claims 2 to 4, wherein the magnet is associated with a pliable area of the medical tube such that the magnet may be readily displaced to the second position.

6. The medical tube of Claim 2, wherein the magnet is within an external chamber attached to the medical tube.

7. The medical tube of Claim 6, wherein a temporary adhesive is provided between the external chamber and the tube.

8. A medical tube comprising a tube or device suitable for insertion into a patient's body and a permanent magnet and a sensing element or device associated with the medical tube.

9. The medical tube of Claim 8, wherein the sensing element or device comprises a pressure sensor, a concentration sensor, a pH sensor, a temperature sensor, a light intensity sensor, an ultrasound image sensor, a video image sensor or a color sensor.

10. A tracheal tube for insertion into the trachea of a patient comprising a tube portion having a distal end and a magnet associated with the tube portion, wherein the magnet is associated with the tube portion at a distance from the distal end such that, when the tube portion is properly inserted into the trachea of the patient, the magnet is positioned immediately posterior to the cricothyroid ligament.

11. The tracheal tube of Claim 10, wherein the distance ranges from about 4 cm to about 6 cm from the distal end.

12. The tracheal tube of Claim 10, wherein the distance is about 5 cm from the distal end.

13. The tracheal tube of any one of Claims 10 to 12, wherein the magnet is a solid magnet associated with an external surface of the medical tube.

14. The tracheal tube of any one of Claims 10 to 12, wherein the magnet is a hollow magnet.

15. The tracheal tube of any one of Claims 10 to 14, wherein the tracheal tube is an endotracheal tube.

16. The tracheal tube of any one of Claims 10 to 14, wherein the tracheal tube is a nasotracheal tube.

17. The tracheal tube of any one of Claims 10 to 16, wherein the magnet is removably associated to the tracheal tube.

18. The tracheal tube of any one of Claims 10 to 16, wherein the magnet is permanently affixed to the tube portion.

19. The tracheal tube of any one of Claims 10 to 18, wherein the magnet is associated with the tube portion such that its magnetic dipole is parallel to a longitudinal axis of the tracheal tube.

20. The tracheal tube of any one of Claims 10 to 18, wherein the magnet is associated with the tube portion such that its magnet dipole is parallel to a transverse axis of the tracheal tube.

21. The tracheal tube of any one of Claims 10 to 20, wherein the tube portion has an anterior side and a posterior side, and wherein the magnet is associated with the anterior side of the tube portion.

22. A medical tube comprising a tube or device suitable for insertion into a patient's body and a permanent magnet associated with the medical tube, wherein the magnet is associated with an exterior surface of the medical tube.

23. The medical tube of Claim 22, wherein the magnet is associated with the exterior surface at the distal end of the medical tube.

24. The medical tube of Claim 22, wherein the magnet is associated with the exterior surface along the length of the medical tube.

25. The medical tube of any one of Claims 22 to 24, wherein the magnet is a non-rigid magnet.

26. The medical tube of any one of Claims 22 to 25, wherein the medical tube has an opening along its length.
